# FASCICULE DE BREVET EUROPEEN

(11) **EP 3 004 976 B1**
(45) Date de publication et mention de la délivrance du brevet: **27.01.2021**
(21) Numéro de dépôt: 14733239.9
(22) Date de dépôt: 03.06.2014
(51) Int. Cl.: G02C 13/00, A61B 3/11, A61B 3/113

(54) **PROCEDE DE DETERMINATION D'AU MOINS UNE VALEUR D'UN PARAMETRE DE PERSONNALISATION D'UN EQUIPEMENT DE COMPENSATION VISUELLE**
VERFAHREN ZUR BESTIMMUNG VON MINDESTENS EINEM WERT EINES PARAMETERS ZUR ANPASSUNG EINER VORRICHTUNG ZUR VISUELLEN KOMPENSATION
METHOD FOR DETERMINING AT LEAST ONE VALUE OF A PARAMETER FOR CUSTOMISING A VISUAL COMPENSATION DEVICE

(30) Priorité: 07.06.2013 FR 1301309
(43) Date de publication de la demande: 13.04.2016
(73) Titulaire: Essilor International, 94220 Charenton-le-Pont (FR)
(72) Inventeur: HADDADI, Ahmed, F-94227 Charenton-le-Pont Cedex (FR); BERTHEZENE, Marie-Anne, F-94227 Charenton-le-Pont Cedex (FR); POULAIN, Isabelle, F-94227 Charenton-le-Pont Cedex (FR); PETIGNAUD, Cécile, F-94227 Charenton-le-Pont Cedex (FR); LEVRAUD, Loïc, F-94227 Charenton-le-Pont Cedex (FR); GAYAT, Sébastien, F-94227 Charenton-le-Pont Cedex (FR); DIVO, Fabien, F-94227 Charenton-le-Pont Cedex (FR); ROUSSEAU, Beanjamin, F-94227 Charenton-le-Pont Cedex (FR)
(74) Mandataire: Jacobacci Coralis Harle
(86) Numéro de dépôt international: PCT/FR2014/051309
(87) Numéro de publication internationale: WO 2014/195623

(56) Documents cités:
- WO-A2-2011/067478
- AU-A1- 2010 249 222
- DE-A1-102011 009 646
- FR-A1- 2 971 861
- US-A1- 2004 189 935
- US-B1- 6 792 401
- "IMPRESSIONIST - DAS 4-IN-1 INTEGRIERTE SERVICE-TERMINAL VON RODENSTOCK", DEUTSCHE OPTIKER ZEITUNG, XX, DE, 1 January 2006 (2006-01-01), pages 56-61, XP000962763,

## Description

### DOMAINE TECHNIQUE AUQUEL SE RAPPORTE L'INVENTION

La présente invention concerne de manière générale le domaine de l'optométrie.

Elle concerne plus particulièrement un procédé d'optométrie pour déterminer une valeur d'au moins un paramètre de personnalisation d'un équipement de compensation visuelle d'un utilisateur. Dans le présent document, on entend typiquement par équipement de compensation visuelle, des lunettes de compensation visuelle comprenant une monture de lunettes et au moins une lentille mono- ou multi-focale. Des mesures d'optométrie visant à déterminer un ou plusieurs paramètres de personnalisation sont nécessaires pour concevoir et fabriquer les faces de réfraction de la lentille de compensation et pour adapter ces lentilles sur la monture choisie par l'utilisateur, en fonction des conditions d'utilisation prévues telles que vision de près, vision de loin et/ou vision au cours d'une activité spécifique.

### ARRIERE-PLAN TECHNOLOGIQUE

On connaît des procédés classiques d'optométrie, ou plus généralement de prise de mesure de paramètres de personnalisation d'un équipement de compensation visuelle, dans lesquels un optométriste utilise un ou plusieurs appareils d'optométrie dédiés à l'acquisition de mesures précises en présence d'un utilisateur. Plus particulièrement, un procédé classique d'optométrie vise à déterminer des paramètres de personnalisation pour pouvoir adapter le montage des lentilles de compensation sur une monture particulière en fonction de l'individu. Les paramètres de personnalisation comprennent notamment des paramètres relatifs à la morphologie d'un individu, tels que l'écart inter-pupillaire, la forme du visage de l'individu, des paramètres de posture, tels que l'angle de tête (roulis, tangage, lacet) par rapport à une ligne verticale dans une posture déterminée, des paramètres de comportement dynamique pour passer d'une posture de vision à une autre posture et aussi des paramètres géométriques ou physionomiques relatifs au visage du porteur et à une monture de lunettes particulière, tels qu'un paramètre de centrage ou de positionnement d'une lentille de compensation sur une monture de lunettes par rapport au centre de rotation optique d'un œil, la hauteur des yeux par rapport au bord inférieur de la monture, le vertex (distance entre l'œil et la face interne du verre de lunettes), l'angle de galbe de la monture ou encore l'angle pantoscopique. L'angle pantoscopique d'une lentille est défini comme étant l'angle entre le plan de maintien de la lentille de compensation dans la monture et un plan vertical, dans une posture naturelle de vision de l'individu.

Certains paramètres de personnalisation relatifs à la morphologie d'un individu sont généralement mesurés dans des conditions où l'individu ne porte pas de lunettes, tandis que d'autres paramètres de personnalisation sont déterminés dans des conditions où l'individu est équipé d'une monture de lunettes ou de lunettes de compensation finies pour l'ajustage sur le visage de l'individu après fabrication.

Les procédés classiques d'optométrie impliquent généralement des interactions entre l'optométriste, l'utilisateur, le(s) appareil(s) d'optométrie et l'équipement de compensation, pendant un laps de temps limité, pour parvenir à définir les caractéristiques d'un équipement de compensation visuelle parfaitement adapté à la morphologie et aux besoins de compensation visuelle de l'utilisateur.

Ces procédés d'optométrie sont complexes et nécessitent la présence de l'utilisateur auprès de l'optométriste et des appareils d'optométrie spécifiques. De plus, certaines mesures ne sont pas réalisables dans un laps de temps limité à quelques dizaines de minutes. En particulier, les procédés classiques d'optométrie ne prennent généralement pas en compte le comportement de l'utilisateur en mouvement, tel qu'un mouvement combiné de la tête et du regard pour passer d'une posture en vision de loin à une posture en vision de près. Ces procédés classiques n'intègrent pas de mesures cinétiques de vision, par exemple pour tenir compte des positions de vision intermédiaires entre la vision de loin et la vision de près.

Depuis quelques années, sont apparues des offres de vente de lunettes en ligne. Le fournisseur propose à l'utilisateur une gamme de montures de lunettes, associées à un choix de lentilles définies en fonction d'une prescription de compensation visuelle préalablement établie par un spécialiste en ophtalmologie. On propose également un procédé simplifié pour déterminer un paramètre de personnalisation au moyen d'une caméra d'ordinateur ou de tablette numérique et d'un logiciel de traitement d'image. Ce procédé permet à un utilisateur d'évaluer un paramètre de personnalisation à partir de son ordinateur ou d'une tablette numérique, sans avoir à se déplacer chez un optométriste. La personnalisation de ce type de lunettes est généralement limitée à l'estimation approximative d'un petit nombre de paramètres tels que l'écart inter-pupillaire. Ainsi, le document WO2011/067478 (JMC Delort) décrit un procédé et dispositif de prise de mesure pour la réalisation de lunettes de vue, sans contact direct, physique, entre le porteur et le professionnel qui prépare les lunettes. Ce procédé se base sur la prise de mesures et de vues photographiques par le client équipé d'un dispositif de mesure et sur l'envoi à distance de ces prises de vues photographiques et de ces mesures. Un autre document pertinent dans ce domaine technique est AU2010249222A1.

Cependant, la qualité des lunettes de compensation visuelle ainsi obtenues est généralement bien inférieure à celle des lunettes définies et ajustées suivant un procédé classique d'optométrie. On constate notamment des défauts de centrage des lentilles et des erreurs sur les écarts inter-pupillaires. Ces défauts proviennent en particulier d'erreurs sur les mesures de paramètres de personnalisation. D'une part, l'utilisateur ne dispose pas de moyens pour contrôler son positionnement par rapport à la caméra : la distance entre la caméra et l'individu est inconnue, si bien qu'une mise à l'échelle de l'image est nécessaire. D'autre part, la caméra qui équipe un ordinateur ou une tablette numérique présente généralement des distorsions d'image importantes. De ce fait, la détermination d'un paramètre de personnalisation de lunettes de compensation visuelle au moyen d'une caméra intégrée est généralement peu fiable. Ces défauts peuvent induire une mauvaise compensation visuelle et/ou un inconfort visuel pour l'utilisateur. De plus, l'utilisateur est amené, dans certains cas, à fixer la caméra précisément (pour obtenir des mesures précises, telles que les demi-écarts pupillaires, la hauteur d'œil, etc...). Or, l'utilisateur peut difficilement se positionner correctement, sans support, car il ne peut observer simultanément la caméra et les consignes de l'écran. En effet, s'il regarde l'écran, il modifie sa posture vis à vis de la caméra. La qualité des mesures se trouve affectée du défaut de positionnement.

Enfin, la caméra et l'écran d'un ordinateur, d'une tablette ou d'un téléphone sont généralement solidaires, ce qui complique les mesures de certains paramètres, par exemple l'angle pantoscopique sur une vue de profil de l'utilisateur, puisque dans cette posture, l'écran est en dehors du champ de vision de l'utilisateur.

Il existe un besoin pour un procédé de détermination d'au moins un paramètre de personnalisation d'un équipement de compensation visuelle, ce procédé pouvant être effectué à distance, par exemple chez un utilisateur, et ce procédé fournissant des mesures fiables d'au moins un paramètre de personnalisation.

Il existe un besoin pour un procédé de détermination d'au moins un paramètre de personnalisation d'un équipement de compensation visuelle, ce procédé s'étendant sur un laps de temps de plusieurs dizaines de minutes, ou même sur plusieurs heures, afin de prendre en compte certains paramètres de personnalisation, qui ne peuvent pas être mesurés dans un laps de temps limité à une ou quelques dizaines de minutes et sans impliquer de surcoût pour l'utilisateur. Il est souhaitable par exemple de moyenner des mesures d'écart pupillaire, de hauteur etc., provenant de plusieurs séquences d'acquisitions étalées dans le temps pour améliorer la précision des mesures.

### OBJET DE L'INVENTION

Afin de remédier à l'inconvénient précité de l'état de la technique, la présente invention propose un procédé d'optométrie dans lequel les mesures sont effectuées à partir d'un terminal de capture d'image et validées à distance après contrôle selon la revendication 1.

Le procédé de l'invention permet de valider une image capturée à distance au moyen d'un terminal et de déterminer une valeur d'au moins un paramètre de personnalisation d'un équipement de compensation visuelle.

Le procédé évite ainsi l'utilisation d'appareils d'optométrie spécifiques coûteux et qui nécessitent un déplacement de l'utilisateur auprès de ces appareils d'optométrie. Le procédé permet néanmoins d'obtenir des valeurs de paramètres de personnalisation validées par une personne habilitée qui assiste l'utilisateur dans la prise de mesure à distance.

Avantageusement, ledit au moins un paramètre de personnalisation comporte l'un des paramètres suivants :
- un paramètre de forme du visage de l'utilisateur,
- un paramètre de posture de l'utilisateur,
- un paramètre de comportement dynamique de l'utilisateur en mouvement,
- un paramètre de centrage d'un verre de compensation sur une monture de lunettes,
- un paramètre de positionnement des centres pupilles ou des CRO des yeux par rapport à un référentiel lié à la monture,
- un paramètre de vertex, par exemple une distance verre-œil,
- un paramètre de positionnement ou d'inclinaison de la monture ou de l'équipement ou du verre par rapport à un référentiel visage ou environnement, par exemple un angle de galbe ou un angle pantoscopique ;
- des paramètres d'ajustage de la monture par rapport à un référentiel visage.

Plus précisément, un ou plusieurs paramètre(s) de forme du visage peuvent permettre de déterminer la forme générale du visage parmi un ensemble de formes prédéfinies telles que : carré, rond, rectangulaire, ovale... et/ou des paramètres d'ajustage d'une monture de lunettes. Un paramètre de posture de l'utilisateur permet de déterminer une posture parmi un ensemble prédéfini de postures tel que : posture droite de vision au loin, posture de lecture en vision de près, posture de travail sur un ordinateur. Un paramètre de comportement de l'utilisateur en mouvement représente un paramètre parmi un ensemble de mouvements prédéfinis tels que : mouvement pour passer d'une première posture à une autre posture, mouvement de hochement de tête d'avant en arrière, ou un mouvement de pivotement de la tête autour d'un axe vertical... Un paramètre de centrage permet de déterminer un paramètre de positionnement d'une lentille relativement à une monture de lunettes, incluant en particulier un paramètre de positionnement d'une lentille par rapport une monture.

D'autres caractéristiques non limitatives et avantageuses de procédé conforme à l'invention sont les suivantes :
- à l'étape d), le centre de téléassistance délivre une instruction de validation si ladite au moins une image ou séquence vidéo est satisfaisante pour au moins contribuer à la détermination de la valeur du paramètre de personnalisation recherché, ou respectivement délivre une instruction de correction dans le cas contraire ;
- à l'étape d), le centre de téléassistance délivre une instruction de correction d'une position de centrage des yeux devant l'appareil de capture d'image, d'une posture de l'utilisateur ou d'une séquence de mouvements de l'utilisateur relativement au capteur d'image de l'appareil de capture d'image ou de suivi d'un protocole présenté par le terminal et à exécuter par le porteur pour obtenir une image validée ;

- au cours de la réitération des étapes a) à e), les instructions de correction délivrées par le centre de téléassistance sont telles que les messages délivrés à l'utilisateur le conduisent à adopter une série de fixations oculaires ou de poursuites oculaires et/ou une série de postures dans lesquelles le plan de Francfort et/ou le plan sagittal de l'utilisateur sont orientés suivant un angle prédéfini relativement à l'axe optique de l'appareil de capture d'image ou à suivre et à exécuter un protocole présenté par le terminal pour obtenir une image validée;
- l'étape a) comprend l'enregistrement vidéo par l'appareil de capture d'image d'une séquence vidéo de l'utilisateur dans la série de postures validée par téléassistance ;
- la série de postures de l'utilisateur devant l'appareil de capture d'image est telle que le plan de Francfort et le plan sagittal de l'utilisateur forment avec l'axe optique de l'appareil de capture d'image un angle inférieur à un seuil prédéfini et dans lequel à l'étape g), on calcule un écart inter-pupillaire ou un demi- écart pupillaire ;
- la série de fixations de l'utilisateur devant l'appareil de capture d'image est telle que la direction de regard forme avec la direction de l'objet fixé un angle inférieur à un seuil prédéfini (facultatif) ;
- à l'étape d), la téléassistance audio-vidéo comprend la communication à l'utilisateur d'instructions de remise à l'échelle comportant la capture d'au moins une image d'un élément de dimension connue, de préférence disposé à proximité des yeux de l'utilisateur, par exemple un clip fixé sur la monture, ou la capture d'une image représentative de l'écart inter-pupillaire d'un utilisateur dont l'écart inter-pupillaire est préalablement connu, ou une mesure de la distance de lecture connue pour corriger la convergence ou encore la capture d'une image d'une monture ayant au moins une dimension géométrique prédéfinie.

De façon préférée, l'interface entre l'utilisateur et une personne du centre de téléassistance se fait par un lien direct. Ce lien direct permet notamment d'assister l'utilisateur permettre dans des cas particuliers, relativement complexes qui ne sont pas facilement automatisables. Ce lien direct permet en outre d'apporter un service au client pour le rassurer dans sa démarche et valider les différentes étapes.

Avantageusement, l'étape d) de téléassistance audio-vidéo comporte les sous étapes suivantes :
- une étape de télétransmission sur l'interface graphique du terminal de l'utilisateur d'un flux vidéo ou d'une image connue par le centre de téléassistance,
- une étape de capture d'image du visage ou d'au moins un œil de l'utilisateur en réponse à ce flux vidéo ou cette image connue, et/ou
- une étape de contrôle de la cohérence de cette réponse du visage ou d'au moins un œil de l'utilisateur à la télétransmission dudit flux vidéo ou de ladite image connue par le centre de téléassistance.

Avantageusement :
- l'étape g) comporte une vérification d'une valeur d'un paramètre géométrique d'ajustage d'une monture de lunettes par comparaison de ladite valeur déterminée avec une valeur de référence ;
- l'étape g) comporte une mesure de la distance de lecture ou de l'abaissement de la tête ou de la direction du regard.

Dans un mode de réalisation particulier, à l'étape a), lors de la capture d'au moins une image ou séquence vidéo, l'utilisateur est équipé d'une monture de lunettes ;
- les messages émis par le terminal comportent un guidage de l'utilisateur dans au moins une première posture dans laquelle le plan sagittal est aligné avec l'axe de l'appareil de capture d'image et au moins une deuxième posture dans laquelle le plan sagittal forme un angle non nul par rapport à l'axe de l'appareil de capture d'image ;
- les images ou séquences vidéo sont validées dans ces deux postures.

Avantageusement, le procédé comprend une étape supplémentaire de sélection d'une langue pour l'utilisateur parmi une pluralité de langues, et un ensemble de messages de guidage de l'utilisateur et de validation étant enregistrées, chaque message est rattaché informatiquement à son implémentation audio-visuelle dans chacune des langues de la pluralité de langues, le message étant délivré par le terminal à l'utilisateur en correspondance avec l'instruction délivrée par le centre de téléassistance et dans la langue sélectionnée par l'utilisateur.

De façon avantageuse, l'étape c) de traitement, par le centre de téléassistance (22), de ladite au moins une image ou séquence vidéo transmise à l'étape b) et/ou l'étape d) de télécommunication du centre de téléassistance (22) au terminal électronique (2) de l'utilisateur de ladite instruction de correction ou de validation, est effectuée par un système automatisé ou par un opticien disposant d'une signature numérique.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement un exemple de mise en œuvre du procédé de l'invention ;
- la figure 2 est une vue de profil de l'individu portant à la main un objet dans une posture en vision de près et d'un terminal selon un autre exemple de mise en œuvre de l'invention ;

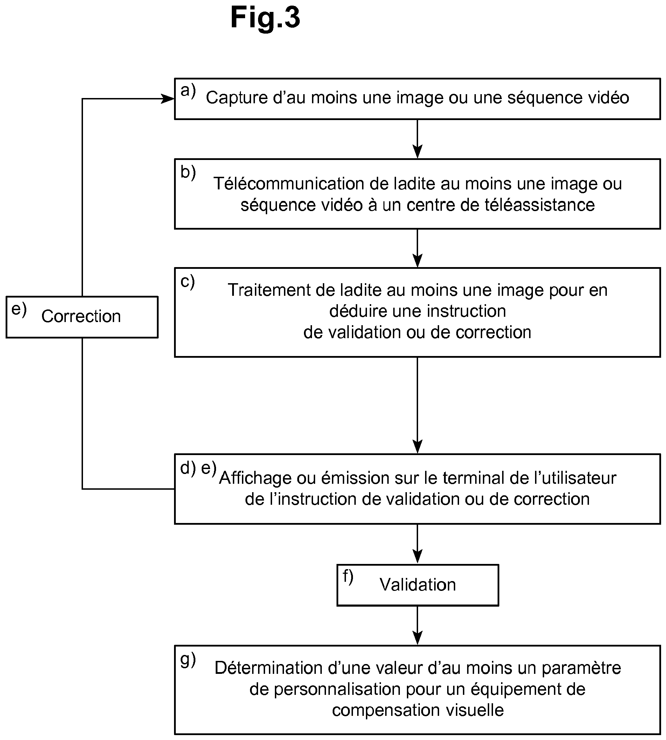

- la figure 3 représente un diagramme des étapes d'un procédé selon un mode de réalisation de l'invention.

### Dispositif

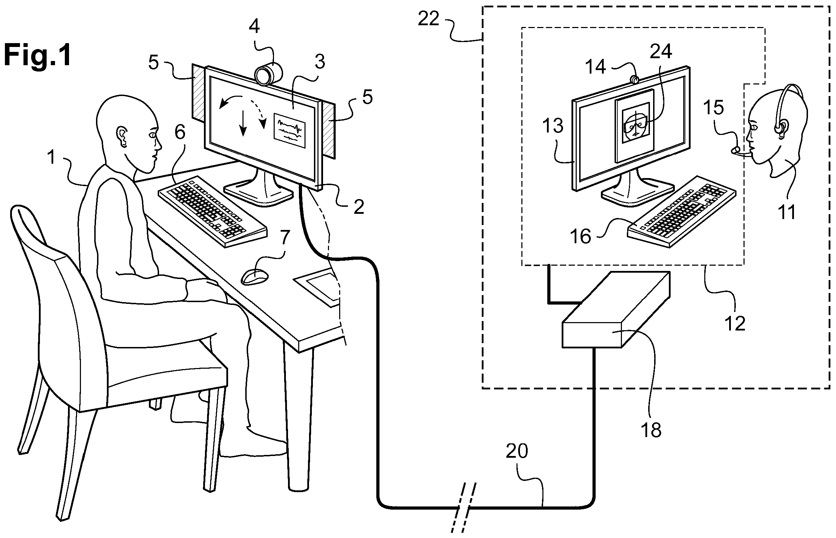

Sur la figure 1, on a représenté schématiquement les éléments d'un dispositif mis en œuvre pour déterminer ou mesurer au moins une valeur d'un paramètre de personnalisation d'un équipement de compensation visuelle destiné à un individu 1 ou utilisateur.

Le dispositif comporte d'une part un terminal audio-visuel 2 et d'autre part un terminal de contrôle 12 situés à distance l'un de l'autre et reliés à un système de traitement informatique 18 par des moyens de télécommunication 20 tels que le réseau internet ou un autre type de réseau.

Le terminal audio-visuel 2 de l'utilisateur comporte un écran de visualisation 3, une caméra 4, un périphérique audio tel que des haut-parleurs 5 ou un casque audio. Le terminal audio-visuel 2 peut consister en un ordinateur muni de moyens d'interface tels qu'un clavier d'ordinateur 6 ou une souris 7 ou une tablette numérique à écran tactile et/ou à commande vocale.

Le terminal de contrôle 12 comporte un écran de visualisation 13, des moyens d'interface tels qu'un clavier d'ordinateur 16 ou un microphone 15. Le terminal de contrôle 12 peut comprendre l'interface du système informatique 18.

De façon avantageuse, le terminal de contrôle 12 est situé dans un centre de téléassistance 22, où une personne habilitée 11 a la possibilité d'interagir avec les différentes interfaces du terminal de contrôle. On entend par centre de téléassistance un ensemble de moyens techniques et humains comprenant au moins un terminal de contrôle 12 et une personne habilitée 11 à contrôler ce terminal de contrôle 12.

Le centre de téléassistance 22 peut comporter un ou plusieurs terminaux de contrôle. Une personne habilitée 11 peut piloter un ou plusieurs terminaux de contrôle 12. Plusieurs terminaux de contrôle 12 peuvent être reliés à un même système de traitement informatique 18. Un centre de téléassistance 22 peut regrouper plusieurs personnes habilitées 11 pour piloter plusieurs terminaux de contrôle 12.

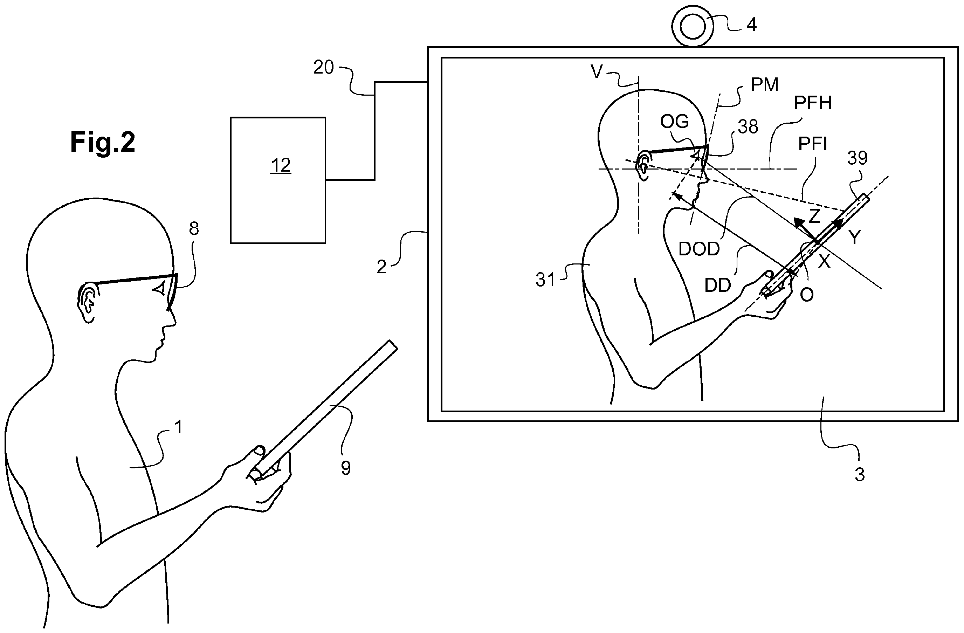

Sur la figure 2, on a représenté un autre exemple de mise en œuvre d'un terminal audio-visuel 2 et d'autre part un terminal de contrôle 12 reliés à un système de traitement informatique 18 par des moyens de télécommunication 20.

### Procédé

L'individu 1, dont on cherche à déterminer au moins un paramètre de personnalisation, est installé devant un terminal électronique audio-visuel 2. Avantageusement, l'individu 1 est placé dans son environnement habituel. De manière alternative, le terminal audio-visuel 2 peut être situé dans un point de vente d'un réseau d'opticiens et relié au terminal de contrôle 12 via un réseau de télécommunication dédié 20.

Dans une alternative, le terminal électronique audio-visuel 2 et le terminal de contrôle 12 sont intégrés dans un même châssis tel qu'une cabine de photomaton. Dans ce cas, le terminal de contrôle 12 comporte des moyens de traitement entièrement automatisés pour l'acquisition des images et pour la communication audio-visuelle avec l'individu 1.L'individu 1 se place devant la caméra 4 du terminal audio-visuel 2. A titre d'exemple illustratif, on cherche à mesurer l'écart inter-pupillaire de l'individu. On entend par écart inter-pupillaire (IPD « inter-pupillary distance »), l'écart entre les centres des pupilles des deux yeux et par demi-écart pupillaire (1/2-IDP) l'écart du centre de la pupille d'un œil par rapport au nez ou au plan sagittal.

L'individu 1 exécute un programme informatique destiné à acquérir au moins une image ou une séquence vidéo au moyen de la caméra 4.

Le démarrage du programme informatique peut comprendre une première étape facultative de sélection d'une langue pour l'utilisateur parmi une pluralité de langues de manière à ce que les messages audio-visuels émis par le terminal soient exprimés dans la langue sélectionnée par l'utilisateur.

Une personne habilitée 11 à contrôler l'acquisition des données de mesure se tient à proximité du terminal de contrôle 12. De préférence, la personne habilitée 11 est une personne qualifiée en optométrie ou un opticien qui est qualifié pour évaluer la qualité des images capturées en vue d'une mesure d'optométrie. De façon encore préférée, la personne habilitée 11 est qualifiée pour apposer une signature ou un sceau de certification de manière à valider officiellement les valeurs de paramètres de personnalisation déterminées par le procédé de mesure. La langue utilisée sur le terminal de contrôle 12 n'est pas nécessairement la même langue que la langue sélectionnée par l'utilisateur, le système informatique 18 étant configuré pour permettre la communication entre le terminal audio-visuel 2 et le terminal de contrôle 12 dans une pluralité de langues respectivement pour le terminal de contrôle et pour le terminal électronique de l'utilisateur.

Le terminal de contrôle permet à la personne habilitée 11 par exemple de visualiser une image 24 ou une séquence vidéo capturée par la caméra 4 du terminal audio-visuel 2. Le système informatique 18 comporte des moyens de traitement numérique pour extraire d'une image ou d'une séquence d'image validée par la personne habilitée 11 au moins une valeur d'un paramètre de personnalisation.

La personne habilitée 11 peut interagir avec l'utilisateur 1 en saisissant une ou plusieurs instructions sur le terminal de contrôle 12 pour déclencher à distance l'émission de messages audio et/ou visuels sur le terminal audio-visuel 2.

Une base de données est chargée sur le système informatique 18 ou localement sous forme de « plug-in » sur le terminal électronique 2. Cette base de données comprend une série d'instructions de commande, chaque instruction de commande étant associée informatiquement à un message destiné à être représenté sur le terminal 2. De façon particulièrement avantageuse, chaque message est rattaché informatiquement à son implémentation audio-visuelle dans chacune des langues de la pluralité de langues proposées à l'utilisateur, le message étant délivré par le terminal 2 à l'utilisateur 1 en correspondance avec l'instruction délivrée par le centre de téléassistance et dans la langue sélectionnée par l'utilisateur 1. De manière analogue, chaque instruction de la série d'instructions de commande est rattachée informatiquement à sa traduction et son implémentation dans chacune des langues de la pluralité de langues disponibles sur le terminal de contrôle 12.

Par exemple, la personne habilitée saisit une instruction de validation sur un bouton affiché sur l'écran 13 du terminal de contrôle 12 ce qui déclenche l'affichage d'un message visuel, par exemple OK, sur l'écran 3 du terminal 2 de l'utilisateur. Dans un autre exemple, une instruction de non-validation déclenche l'émission d'un message de signal d'alerte sonore et l'affichage d'un message visuel sur l'écran 3, par exemple sous forme d'un flux vidéo affichant l'image d'une flèche clignotante accompagnée d'un texte court dans la langue sélectionnée par l'utilisateur, le message textuel demandant par exemple à l'utilisateur d'incliner la tête suivant la direction indiquée par la flèche clignotante.

La base de données assure ainsi la correspondance informatique et linguistique entre une instruction saisie par la personne habilitée et un message ou une série de messages émis par le terminal de l'utilisateur. La base de données effectue aussi une traduction automatique du message émis ou affiché dans la langue de l'utilisateur.

Le sens inverse de communication, de l'utilisateur 1 vers le centre de téléassistance 22, est aussi prévu. La base de données comporte à cet effet une série d'instructions de commande utilisateur, chaque instruction de commande utilisateur étant associée informatiquement à un message destiné à être représenté sur le terminal de contrôle 12 du centre de téléassistance 22. De préférence, chaque instruction de commande utilisateur est rattachée informatiquement à son implémentation dans chacune des langues de la pluralité de langues disponibles sur le terminal de contrôle 12.

Par exemple, l'utilisateur peut saisir une instruction ou une question sur son terminal qui déclenche la traduction de cette instruction ou question et l'émission d'un message sur le terminal de contrôle 12 de la personne habilitée. Par exemple, l'utilisateur peut appuyer sur un bouton « ? » qui déclenche l'affichage d'un message « demande d'aide » sur le terminal de contrôle 12, traduit dans la langue disponible pour la personne habilitée.

A titre d'exemple non limitatif, on cherche à mesurer un paramètre d'écart inter-pupillaire de l'individu. L'individu 1 démarre un programme d'acquisition d'image. Une première image capturée est transmise via les moyens de télécommunications 20 au centre de téléassistance 22 ou centre de traitement à distance. Sur l'écran 13 du terminal de contrôle 12 s'affiche l'image capturée 24 ainsi qu'au moins un indicateur pour évaluer la qualité de cette image. L'indicateur de qualité peut être calculé par le système informatique 18 ou être évalué et saisi par la personne habilitée 11.

La personne habilitée 11 est chargée d'évaluer la qualité de l'image capturée et de valider ou non cette image pour une mesure d'écart inter-pupillaire. Le procédé ne repose pas nécessairement sur un ou plusieurs critères de qualité préenregistrés dans le système informatique 18 mais peut aussi reposer sur une évaluation subjective de la qualité de l'image capturée par la personne habilitée 11, en fonction de ses compétences et de son savoir-faire.

A titre d'exemple, supposons que la première image ne convienne pas pour une mesure d'écart inter-pupillaire, pour des raisons de défaut de cadrage de l'image ou d'inclinaison de la tête de l'individu.

La personne habilitée 11 saisit, par exemple via le clavier 16 ou un autre périphérique du terminal de contrôle 12, une instruction de non validation de la première image capturée.

De manière automatique, ou sur une commande de la personne habilitée 11 à distance, le système informatique 18 traite cette instruction de non validation. Plus précisément, le système informatique pointe le champ « message » de la base de données correspondant à l'instruction de non validation et à la langue de l'utilisateur. Le système informatique 18 déclenche la délivrance sur le terminal 2 d'un message audio-visuel correspondant à l'instruction de non validation, le message délivré étant par exemple un message audio via les haut-parleurs 5 et/ou un message visuel affiché sur l'écran de visualisation 3. Le terminal 2 affiche un message visuel et/ou émet un message audio demandant à l'utilisateur de se positionner ou de se déplacer conformément à l'instruction de correction. Avantageusement, la personne habilitée peut guider l'utilisateur 1 pour qu'il modifie sa posture face à la caméra. Les messages visuels peuvent comprendre des messages textuels, des icônes ou des messages vidéo préenregistrés. Suite au repositionnement de l'utilisateur, la caméra 4 capture une deuxième image ou une deuxième séquence vidéo.

La deuxième image capturée est transmise via les moyens de télécommunications 20 au centre de téléassistance 22.

Sur le terminal de contrôle 13 s'affiche la deuxième image capturée ainsi que l'indicateur pour évaluer la qualité de cette image. Si l'indicateur de qualité remplit des critères de qualité, la personne habilitée 11 peut valider la deuxième image. La saisie d'une commande de validation par la personne habilitée 11 est enregistrée dans le système informatique 18. Le système informatique 18 pointe vers le champ de la base de données correspondant à l'instruction de validation et à la langue de l'utilisateur et déclenche la transmission d'un message de validation sur le terminal 2 de l'utilisateur.

Le terminal 2 affiche un message visuel et/ou émet un message audio de manière à informer l'utilisateur 1 de la validation de la capture d'image ou de séquence vidéo.

Dans le cas où la deuxième image capturée ne remplit pas les critères de qualité, le système informatique 18 délivre à nouveau un message de non validation de la deuxième image capturée, et le procédé de capture d'image est réitéré ou abandonné par l'utilisateur.

Le système informatique 18 effectue un traitement numérique de l'image ou de la séquence vidéo capturée et validée pour en extraire la valeur du paramètre de personnalisation recherché.

Dans un exemple de détermination de la forme du visage de l'utilisateur, un traitement d'image est appliqué afin de définir le contour du visage sur l'image et l'associer par exemple à une forme géométrique prédéfinie.

Dans le cas d'une mesure, telle qu'une mesure de distance inter-pupillaire à partir d'une image de la caméra, cette mesure suppose que l'échelle de l'image est connue ou peut être déterminée. Les dimensions de l'image dépendent des propriétés optiques du capteur d'image de la caméra 4, mais aussi de la distance entre la caméra et l'individu.

Avantageusement, le procédé comporte une étape supplémentaire de mise à l'échelle. Cette mise à l'échelle peut être effectuée par la capture d'une image du visage de l'utilisateur avec un objet de dimension connue disposé dans le champ de l'image, de préférence dans le même plan image que le visage de l'individu. Par exemple, la personne habilitée peut demander via l'interface du terminal 2 à l'utilisateur de placer une règle graduée ou un repère de dimension connue sur son front. De manière alternative, si l'utilisateur dispose d'une monture de lunettes ayant au moins une dimension géométrique prédéfinie, l'étape de mise à l'échelle peut comporter une étape de capture d'une image de la monture de lunette faisant apparaître cette dimension géométrique prédéfinie. Le système informatique 18 traite l'image du repère ou de l'objet de dimension connue pour en déduire un facteur de mise à l'échelle de la caméra pour une distance de travail donnée.

Le traitement d'image appliqué à une image ou à une séquence vidéo capturée comporte la détection de l'image d'un point remarquable de l'oreille de l'utilisateur tel que le tragion. Le but de ce traitement d'image est par exemple de déterminer une posture de mesure dans laquelle l'axe de la caméra est situé dans le plan de Francfort de l'utilisateur, le plan de Francfort étant de manière connue en soi le plan anatomique défini par les points d'orbite basse des yeux et les tragions. En pratique, la personne habilitée 11 vérifie que le bas de l'orbite de chaque œil et que les tragions sont alignés avec la pupille de la caméra, ce qui revient à dire qu'il sont vus sur l'image à une même hauteur. Un protocole consiste à ce que la personne habilitée 11 demande à l'utilisateur 1 de faire une première capture d'image de face, puis à ce que l'utilisateur 1 effectue une rotation vers le haut ou le bas de la tête jusqu'à obtenir cet alignement du plan de Francfort parallèlement à l'axe de la caméra. Cet alignement peut éventuellement être suivi d'une rotation de la tête de l'utilisateur 1 vers la droite ou vers la gauche afin de permettre à la personne habilitée 11 de mieux visualiser les tragions et ainsi d'affiner la posture de l'utilisateur 1. En effet, les tragions ne sont généralement pas visibles sur l'image de face mais l'assistant peut estimer leur position grâce aux oreilles. Cette posture de mesure permet d'obtenir une mesure de la hauteur des yeux par rapport au bord inférieur de la monture, à peu près équivalente à une mesure classique en posture orthostatique avec une caméra à la hauteur des yeux. Cette posture de mesure permet notamment d'éviter des postures dans lesquelles les mesures, par exemple de hauteur, seraient complètement fausses du fait d'un angle excessif entre l'axe de la caméra et le plan de Francfort. Ceci peut être appliqué sans monture, pour l'essayage virtuel de monture par exemple et avec un traitement d'image pour en déduire les hauteurs par fit virtuel entre une représentation de la monture et une image du visage de l'utilisateur. Une telle mesure sans monture est certes approximative mais permet néanmoins d'éliminer d'importantes sources d'erreurs. De manière alternative, la mesure peut être réalisée avec une monture réelle pour des résultats de meilleure qualité.

De façon avantageuse, la personne habilitée 11 du centre de téléassistance peut guider l'utilisateur pour qu'il se place dans différentes postures de mesure en fonction des tests et mesures à effectuer.

De manière analogue, il est aussi possible de déterminer une posture de l'utilisateur dans laquelle le plan sagittal est parallèle à l'axe de la caméra.

De façon particulièrement avantageuse, une posture de mesure correspond à une posture dans laquelle le plan sagittal et le plan de Francfort de l'utilisateur sont alignés avec l'axe de la caméra.

A titre d'exemple non limitatifs, on peut mentionner, outre la posture pour mesurer la hauteur détaillée ci-dessus, une posture générale, une posture pour la mesure des demi-écarts pupillaires, une posture ou une série de mouvements pour déterminer la distance verre-œil (DVO) ou encore pour mesurer l'angle pantoscopique des lunettes dans une posture de vision déterminée.

### Posture générale :

La personne habilitée 11 ou un assistant du centre de téléassistance ou un automate vérifie à l'étape c) que le porteur est à une distance de l'écran supérieure à un seuil, par exemple en lui demandant de tendre le bras vers l'écran en le touchant juste ou par exemple en contrôlant que le visage de l'utilisateur 1 occupe une taille minimale dans l'image. La personne habilitée 11 vérifie aussi le bon positionnement d'un élément de mise à l'échelle, si cet élément est présent et/ou que la tête de l'utilisateur 1 n'est pas inclinée de manière à présenter un angle de tangage supérieur à un seuil prédéterminé. Enfin, l'assistant 11 vérifie que l'utilisateur 1 visualise bien la caméra 4, par exemple en lui demandant de pointer la caméra avec son index. Les instructions de remise à l'échelle comprennent par exemple un contrôle de la présence et du positionnement correct de l'élément de remise à l'échelle (clip, monture ou autre élément de dimension connue) et/ou la position du porteur par rapport à la caméra.

### Posture pour la mesure des demi-écarts pupillaires :

L'assistant 11 déclenche l'envoi d'une séquence vidéo qui permet de déterminer si la posture, plus précisément, l'angle d'inclinaison de la tête (headcape) du porteur est adapté ou non à une mesure correcte des demi-écarts pupillaires, par exemple en vérifiant que le visage de l'utilisateur 1 est bien positionné de face par rapport à la caméra 4. Cette étape de mesure peut être automatisée par des algorithmes permettant d'estimer cet angle d'inclinaison de la tête (Headcape, par exemple en utilisant le dispositif Seeing Machine). La personne habilitée 11 valide la posture de mesure soit lorsque les consignes envoyées à l'utilisateur 1 permettent d'atteindre un angle d'inclinaison nul (headcape nul), soit lorsque l'angle d'inclinaison est inférieur à un seuil prédéfini.

### Posture pour déterminer la distance verre-œil (DVO) :

Cette posture est illustrée par exemple sur la figure 2, où le système de traitement est soit un centre de téléassistance avec une personne habilitée 11 soit un système entièrement automatisé.

L'utilisateur 1 porte une monture de lunettes 8 équipée de verres. A titre d'exemple non limitatif, une monture de lunettes 8 portée par l'utilisateur 1 pendant une mesure, peut être une ancienne monture de lunettes ou un équipement de compensation visuelle nouvellement reçu pour contrôle, par exemple de l'angle pantoscopique.

La personne habilitée 11 demande à l'utilisateur 1 de regarder la caméra 4 et de tourner la tête, de manière analogue aux mouvements devant un appareil Visioffice de la société Essilor et lors de l'étape c), la personne habilitée 11 s'assure que la rotation de la tête est suffisante pour réaliser la mesure de ce paramètre.

Dans le cas d'un système automatisé, un programme d'acquisition et de traitement d'image démarre suite à la saisie d'une commande via l'interface audio-visuelle par l'individu. La caméra 8 démarre l'acquisition d'une image ou de préférence d'une séquence d'images. Pendant cette acquisition, l'individu effectue un mouvement de pivotement de la tête de gauche à droite sur une amplitude d'environ ±5 degrés, de manière à ce qu'au moins une image de la séquence d'images corresponde à une vue de profil de l'utilisateur. Le système de traitement d'image effectue un traitement, par exemple en temps réel, pour analyser la posture de l'utilisateur : le système de traitement sélectionne une image correspondant à une vue de profil 31 de l'utilisateur. De façon particulièrement avantageuse, le système automatique de traitement émet des messages audio pour guider l'individu, de manière à ce que l'individu soit à une distance suffisante pour être dans le champ de vision de la caméra, pour qu'il modifie sa posture de tête, l'inclinaison de la tête de gauche à droite et/ou d'avant en arrière.

Ainsi, l'utilisateur est guidé par le système de traitement de manière à ce que ce dernier valide que l'image 31 acquise corresponde effectivement à une vue de profil, par exemple à mieux que ± 5 degrés, et non pas de trois quart par exemple.

La caméra 4 comporte éventuellement un zoom adapté pour capturer une image de profil de l'individu comprenant sa tête et un bras pour permettre d'analyser la posture de l'individu, par exemple en vision de près, lorsque l'individu tient en main un objet 9, tel qu'une livre ou une tablette numérique.

Avantageusement, comme illustré sur la figure 2, le système comporte des moyens de reconnaissance faciale qui permettent de déterminer la position de la tête de l'individu dans l'image acquise, et de certains points particuliers, tels que la position de l'œil droit OD ou gauche OG et de détecter la position des contours des lunettes.

Le système de traitement d'image est aussi configuré pour détecter l'image 39 d'un objet utilisé comme cible du regard pour la vision intermédiaire ou à la vision de près et la position d'un repère (OX, OY, OZ) lié à cet objet 9.

A partir de l'identification de ces points particuliers dans l'image, le système de traitement est par exemple configuré pour déterminer automatiquement dans l'image 31 les positions et orientations d'un plan anatomique de Francfort incliné PFI dans les conditions visuelles de la capture d'image, par exemple en vision de près ou intermédiaire, ou d'un plan anatomique de Francfort horizontal PFH dans les conditions de vision de loin.

De manière connue en soi, le système de traitement est adapté pour compenser l'erreur de parallaxe de l'image 31.

Ainsi, on mesure en outre un paramètre de posture de la tête tel qu'une distance DD, DG entre la tête ou l'un des yeux OD, OG de l'individu et l'objet 9, par exemple plus précisément la distance DD, DG entre le centre de rotation des yeux OD, OG de l'individu et l'objet 9.

La mesure de distances est calibrée par des méthodes connues, par exemple via l'acquisition d'une image d'un objet de taille connue, tel qu'une règle graduée ou une carte de crédit.

On peut également prévoir de déterminer un des paramètres géométriques suivants : un plan de maintien PM d'une lentille, une droite d'observation DOD, DOG reliant l'un des yeux OD, OG de l'individu et un point particulier O de l'objet 9.

Ainsi, on peut également prévoir de mesurer au moins l'un des paramètres suivants : un angle pantoscopique entre le plan de maintien PM sur l'image d'une lentille 38 des lunettes et une droite verticale V dans une posture de vision identifiée. Pour la mesure de certains paramètres de personnalisation, l'utilisateur 1 ne porte ni équipement de compensation visuelle, ni monture d'essayage. Pour d'autres mesures de paramètres de personnalisation (centrage des lentilles par rapport à une monture particulière par exemple), ou pour un contrôle d'ajustage des lunettes après fabrication, il est nécessaire que l'utilisateur porte les lunettes pour la prise d'image ou de séquence vidéo.

Les messages audio-visuels sur le terminal 2 peuvent comprendre des messages pour que l'utilisateur porte les lunettes 8, ou enlève les lunettes 8, ou porte une monture d'essayage dépourvue de lentilles de compensation. Pour des mesures de certaines postures de vision, par exemple de lecture en vision de près, un message audio-visuel peut inviter l'utilisateur à prendre un objet 9 tel qu'un livre.

L'étape de contrôle par téléassistance peut comporter une étape de vérification de la bonne exécution de l'instruction d'enlever ou de porter des lunettes ou de porter une monture sans lentilles de compensation.

Par comparaison avec une mesure classique d'optométrie effectuée sur un appareil d'optométrie par un spécialiste, le procédé de téléassistance autorise des mesures de durée beaucoup plus longue et permet d'obtenir une mesure moyennée sur une série de mesures sans impliquer de surcoût.

La durée de mesure souhaitée par l'utilisateur peut ainsi être définie et saisie par l'utilisateur au démarrage du procédé ou enregistrée par la téléassistance. Un choix des mesures de personnalisation à effectuer en fonction de cette durée peut être proposé ou défini dès le démarrage du procédé. Le choix des mesures de personnalisation à effectuer peut aussi être défini en fonction d'un style de vie détecté ou indiqué, tel que personne travaillant sur ordinateur, alternance de lecture et de vision au loin. Un autre avantage du procédé est de permettre de séparer dans le temps différentes mesures à différents instants de la journée, pour prendre en compte l'évolution des conditions ou paramètres de vision de l'utilisateur.

De façon avantageuse, l'intervention de la personne habilitée 11 n'est pas requise en continu pendant toute la durée du procédé, notamment avant l'étape de capture d'image. L'intervention de la personne habilitée 11 est requise lors de l'étape de validation ou non-validation d'une image ou d'une séquence d'images et éventuellement pour guider un utilisateur afin qu'il modifie sa posture en vue d'une nouvelle acquisition.

Une même personne habilitée 11 a la possibilité de contrôler en parallèle, à partir d'un même terminal de contrôle 12, plusieurs utilisateurs, chaque utilisateur étant situé face à un terminal 11 distinct. Dans ce cas, la personne habilitée 11 porte assistance successivement et de manière intermittente à chacun des utilisateurs.

L'intervention de la personne habilitée 11 peut aussi être requise après traitement d'image par le système informatique et détermination du ou des paramètres de personnalisation recherchés, en fin de protocole pour la validation officielle et/ou la signature numérique des paramètres de personnalisation déterminés par le système informatique. La téléassistance remplit ainsi le rôle d'un opticien à domicile.

De façon avantageuse, une base de données permet d'enregistrer les valeurs de paramètres de personnalisation associées à un utilisateur et à des conditions de mesure définies.

Un opticien de réseau ou un fabriquant de lentilles de compensation visuelle peut ensuite récupérer ces résultats de mesure de paramètre de personnalisation validées par une personne habilitée pour les exploiter afin de lancer la fabrication de l'équipement de compensation visuel adapté aux besoins de l'utilisateur. Les valeurs de paramètres de personnalisation peuvent à cet effet être complétées par d'autres mesures, telles que des mesures opthalmologiques d'un individu comprenant notamment les valeurs de courbure, d'axe et de cylindre de compensation visuelle requises.

Le procédé de téléassistance permet de déterminer au moins une valeur d'un paramètre de personnalisation dans une phase d'avant-vente contrôlée. Ce procédé de téléassistance se traduit par un gain de temps pour l'opticien.

Le procédé peut aussi s'appliquer après la fabrication de lunettes de compensation pour un contrôle de l'ajustage des lunettes pour l'utilisateur.

## Revendications

1. Procédé de détermination d'au moins une valeur d'un paramètre de personnalisation d'un équipement de compensation visuelle d'un utilisateur (1) équipé d'un terminal électronique (2) comprenant une interface graphique (3), un appareil de capture d'image (4) comprenant un seul capteur d'image et des moyens audio-visuels de communication (5), le procédé comportant les étapes suivantes :
a) capture d'au moins une image ou séquence vidéo de l'utilisateur au moyen de l'appareil de capture d'image (4) ;
b) télécommunication de ladite au moins une image ou séquence vidéo capturée à l'étape a), à un centre de téléassistance (22) distant du terminal électronique (2), le centre de téléassistance (22) comprenant au moins un terminal de contrôle (12) ;
c) traitement de contrôle, par le centre de téléassistance (22), de ladite au moins une image ou séquence vidéo transmise à l'étape b), pour en déduire une instruction de correction ou de validation de l'image ou séquence vidéo capturée, compte tenu d'une position, d'une posture ou d'une séquence de mouvements de l'utilisateur devant l'appareil de capture d'image ;
d) télécommunication du centre de téléassistance (22) au terminal électronique (2) de l'utilisateur de ladite instruction de correction ou de validation;
e) affichage ou émission par le terminal électronique (2) d'un message audio ou vidéo informant l'utilisateur de la validation de la capture d'image ou séquence vidéo ou demandant à l'utilisateur de se positionner ou de se déplacer par rapport à l'appareil de capture d'image conformément à l'instruction de correction, l'étape e) comprenant un protocole d'alignement du plan de Francfort de la tête de l'utilisateur avec l'axe de l'appareil de capture d'image, le protocole consistant à ce qu'une personne habilitée du centre de téléassistance demande à l'utilisateur de faire une première capture d'image de face puis comportant une demande de rotation de la tête de l'utilisateur autour d'un axe horizontal jusqu'à obtenir un alignement du plan de Francfort parallèlement à l'axe de l'appareil de capture d'image puis comportant une demande de rotation de la tête de l'utilisateur vers la droite ou vers la gauche pour permettre à la personne habilitée de visualiser les tragions et de déterminer une posture de mesure dans laquelle l'axe de l'appareil de capture d'image est situé dans ledit plan de Francfort ;
f) réitération des étapes précédentes pour suivre et exécuter le protocole d'alignement du plan de Francfort parallèlement à l'axe de l'appareil de capture d'image jusqu'à l'obtention d'une image ou séquence vidéo validée dans ladite posture de mesure ;
g) détermination d'au moins une valeur dudit paramètre de personnalisation en fonction de ladite au moins une image ou séquence vidéo capturée et validée dans ladite posture de mesure, ledit au moins un paramètre de personnalisation comportant un paramètre d'écart inter-pupillaire ou de demi-écart pupillaire droit ou gauche.

2. Procédé selon la revendication 1, dans lequel dans lequel ledit au moins un paramètre de personnalisation comporte l'un des paramètres suivants :
- un paramètre de forme du visage de l'utilisateur,
- un paramètre de posture de l'utilisateur,
- un paramètre de comportement dynamique de l'utilisateur,
- un paramètre de centrage d'un verre de compensation sur une monture de lunettes,
- un paramètre de positionnement des centres pupilles ou des CRO des yeux par rapport à un référentiel lié à la monture,
- un paramètre de vertex, par exemple une distance verre-œil
- un paramètre de positionnement ou d'inclinaison de la monture ou de l'équipement ou du verre par rapport à un référentiel visage ou environnement, par exemple un angle de galbe ou un angle pantoscopique,
- des paramètres d'ajustage de la monture par rapport à un référentiel visage.

3. Procédé selon l'une des revendications précédentes, dans lequel à l'étape d), le centre de téléassistance (22) délivre une instruction de validation si ladite au moins une image ou séquence vidéo est satisfaisante pour au moins contribuer à la détermination de la valeur du paramètre de personnalisation recherché, ou respectivement délivre une instruction de correction dans le cas contraire.

4. Procédé selon l'une des revendications précédentes, dans lequel à l'étape d), le centre de téléassistance (22) délivre une instruction de correction d'une position de centrage des yeux devant l'appareil de capture d'image, d'une posture de l'utilisateur ou d'une séquence de mouvements de l'utilisateur relativement au capteur d'image de l'appareil de capture d'image ou de suivi d'un protocole présenté par le terminal et à exécuter par le porteur pour obtenir une image validée.

5. Procédé selon l'une des revendications précédentes, dans lequel :
- au cours de la réitération des étapes a) à e), les instructions de correction délivrées par le centre de téléassistance (22) sont telles que les messages délivrés à l'utilisateur le conduisent à adopter une série de fixations oculaires ou de poursuites oculaires et/ou une série de postures dans lesquelles le plan de Francfort et/ou le plan sagittal de l'utilisateur sont orientés suivant un angle prédéfini relativement à l'axe optique de l'appareil de capture d'image ou à suivre et à exécuter un protocole présenté par le terminal pour obtenir une image validée ;
- l'étape a) comprend l'enregistrement vidéo par l'appareil de capture d'image (4) d'une séquence vidéo de l'utilisateur dans la série de postures validée par téléassistance.

6. Procédé selon la revendication précédente, dans lequel la série de postures de l'utilisateur devant l'appareil de capture d'image (4) est telle que le plan de Francfort et le plan sagittal de l'utilisateur forment avec l'axe optique de l'appareil de capture d'image un angle inférieur à un seuil prédéfini et dans lequel à l'étape g), on calcule un écart inter-pupillaire ou un demi- écart pupillaire.

7. Procédé selon l'une des revendications précédentes, dans lequel à l'étape d), la téléassistance audio-vidéo comprend la communication à l'utilisateur d'instructions de remise à l'échelle comportant la capture d'au moins une image d'un élément de dimension connue, de préférence disposé à proximité des yeux de l'utilisateur, ou la capture d'une image représentative de l'écart inter-pupillaire d'un utilisateur dont l'écart inter-pupillaire est préalablement connu, ou une mesure de la distance de lecture connue pour corriger la convergence ou encore la capture d'une image d'une monture ayant au moins une dimension géométrique prédéfinie.

8. Procédé selon l'une des revendications précédentes, dans lequel d) de téléassistance audio-vidéo comporte les sous étapes suivantes :
- une étape de télétransmission sur l'interface graphique du terminal de l'utilisateur d'un flux vidéo ou d'une image connue par le centre de téléassistance,
- une étape de capture d'image du visage ou d'au moins un œil de l'utilisateur en réponse à ce flux vidéo ou cette image connue.

9. Procédé selon l'une des revendications précédentes, dans lequel l'étape g) comporte une vérification d'une valeur d'un paramètre géométrique d'ajustage d'une monture de lunettes par comparaison de ladite valeur déterminée avec une valeur de référence.

10. Procédé selon l'une des revendications précédentes, dans lequel l'étape g) comporte une mesure de la distance de lecture ou de l'abaissement tête/direction regard.

11. Procédé selon l'une des revendications précédentes, dans lequel :
- à l'étape a) lors de la capture d'au moins une image ou séquence vidéo, l'utilisateur est équipé d'une monture de lunettes ;
- les messages émis par le terminal comportent un guidage de l'utilisateur dans au moins une première posture dans laquelle le plan sagittal est aligné avec l'axe de l'appareil de capture d'image et au moins une deuxième posture dans laquelle le plan sagittal forme un angle non nul par rapport à l'axe de l'appareil de capture d'image ;
- les images ou séquences vidéo sont validées dans ces deux postures.

12. Procédé selon l'une des revendications précédentes comprenant une étape supplémentaire de sélection d'une langue pour l'utilisateur parmi une pluralité de langues, et dans lequel un ensemble de messages de guidage de l'utilisateur et de validation étant enregistrées, chaque message est rattaché informatiquement à son implémentation audio-visuelle dans chacune des langues de la pluralité de langues, le message étant délivré par le terminal à l'utilisateur en correspondance avec l'instruction délivrée par le centre de téléassistance et dans la langue sélectionnée par l'utilisateur.

13. Procédé selon l'une des revendications précédentes dans lequel l'étape c) de traitement, par le centre de téléassistance (22), de ladite au moins une image ou séquence vidéo transmise à l'étape b) et/ou l'étape d) de télécommunication du centre de téléassistance (22) au terminal électronique (2) de l'utilisateur de ladite instruction de correction ou de validation, est effectuée par un système automatisé.

14. Procédé selon l'une des revendications précédentes dans lequel l'étape c) de traitement, par le centre de téléassistance (22), de ladite au moins une image ou séquence vidéo transmise à l'étape b) et/ou l'étape d) de télécommunication du centre de téléassistance (22) au terminal électronique (2) de l'utilisateur de ladite instruction de correction ou de validation, est effectuée par un opticien (11) disposant d'une signature numérique.

## Patentansprüche

1. Verfahren zur Bestimmung mindestens eines Werts eines Parameters zur Personalisierung einer Ausrüstung zur visuellen Kompensation eines Benutzers (1), der mit einem elektronischen Endgerät (2) ausgerüstet ist, das eine grafische Schnittstelle (3), eine Bilderfassungsvorrichtung (4) umfassend einen einzelnen Bildsensor und audiovisuelle Kommunikationsmittel (5) umfasst, das Verfahren aufweisend die folgenden Schritte:
a) Erfassen mindestens eines Bilds oder einer Videosequenz des Benutzers mittels der Bilderfassungsvorrichtung (4);
b) Fernkommunizieren des mindestens einen Bilds oder der mindestens einen Videosequenz, das/der bei Schritt a) erfasst wurde, an ein Fernassistenzzentrum (22), das vom elektronischen Endgerät (2) entfernt ist, das Fernassistenzzentrum (22) umfassend mindestens ein Kontrollendgerät (12);
c) Verarbeiten, zur Kontrolle, des mindestens einen Bilds oder der mindestens einen Videosequenz, das/die bei Schritt b) übertragen wird, durch das Fernassistenzzentrum (22), um daraus eine Anweisung zur Korrektur oder Bestätigung des erfassten Bilds oder der erfassten Videosequenz abzuleiten, unter Berücksichtigung einer Position, eine Haltung oder eine Abfolge von Bewegungen des Benutzers vor der Bilderfassungsvorrichtung;
d) Fernkommunizieren der Korrektur- oder Bestätigungsanweisung vom Fernassistenzzentrum (22) an das elektronische Endgerät (2) des Benutzers;
e) Anzeigen oder Senden, von dem elektronischen Endgerät (2), einer Audio- oder Videonachricht, die den Benutzer über die Bestätigung der Bild- oder Videosequenzerfassung informiert oder den Benutzer bittet, sich entsprechend der Korrekturanweisung bezogen auf die Bilderfassungsvorrichtung zu positionieren oder zu bewegen, der Schritt e) umfassend ein Protokoll zur Ausrichtung der Frankfurter Horizontalen des Kopfes des Benutzers mit der Achse der Bilderfassungsvorrichtung, wobei das Protokoll darin besteht, dass eine befugte Person des Fernassistenzzentrums den Benutzer bittet, eine erste Bilderfassung von vorne vorzunehmen, dann eine Bitte umfasst, dass der Benutzer den Kopf um eine horizontale Achse dreht, bis eine parallele Ausrichtung der Frankfurter Horizontalen mit der Achse der Bilderfassungsvorrichtung erreicht wird, dann eine Bitte umfasst, dass der Benutzer den Kopf nach rechts oder nach links dreht, um es der befugten Person zu gestatten, die Tragionen zu visualisieren und eine Messhaltung zu bestimmen, bei der die Achse der Bilderfassungsvorrichtung in der Frankfurter Horizontalen liegt;
f) Wiederholen der vorhergehenden Schritte zur Befolgung und Ausführung des Protokolls zur parallelen Ausrichtung der Frankfurter Horizontalen mit der Achse der Bilderfassungsvorrichtung, bis ein bestätigtes Bild oder eine bestätigte Videosequenz in der Messhaltung erhalten wird;
g) Bestimmen mindestens eines Werts des Personalisierungsparameters in Abhängigkeit von dem mindestens einen Bild oder der mindestens einen Videosequenz, das/die in der Messhaltung erfasst und bestätigt wurde, wobei der mindestens eine Personalisierungsparameter einen Parameter eines rechten oder linken Interpupillarabstands oder halben Interpupillarabstands umfasst.

2. Verfahren nach Anspruch 1, wobei wobei der mindestens eine Personalisierungsparameter einen der folgenden Parameter umfasst:
- einen Parameter der Gesichtsform des Benutzers,
- einen Parameter der Haltung des Benutzers,
- einen Parameter des dynamischen Verhaltens des Benutzers,
- einen Parameter der Zentrierung eines Kompensationsglases auf einem Brillengestell,
- einen Parameter der Positionierung der Pupillenzentren oder der CRO der Augen bezogen auf ein Bezugssystem in Verbindung mit dem Gestell,
- einen Vertexparameter, zum Beispiel einen Glas-Auge-Abstand,
- einen Parameter der Positionierung oder Neigung des Gestells oder der Ausrüstung oder des Glases bezogen auf ein Gesichts- oder Umgebungsbezugsmodell, zum Beispiel einen Rundungswinkel oder einen pantoskopischer Winkel,
- Einstellungsparameter des Gestells bezogen auf ein Gesichtsbezugssystem.

3. Verfahren nach einem der vorhergehenden Ansprüche, wobei bei Schritt d) das Fernassistenzzentrum (22) eine Bestätigungsanweisung erteilt, wenn das mindestens eine Bild oder die mindestens eine Videosequenz zufriedenstellend ist, um zur Bestimmung des Werts des gesuchten Personalisierungsparameters zumindest beizutragen, oder im gegenteiligen Fall eine Korrekturanweisung erteilt.

4. Verfahren nach einem der vorhergehenden Ansprüche, wobei bei Schritt d) das Fernassistenzzentrum (22) eine Anweisung zur Korrektur einer Zentrierungsposition der Augen vor der Bilderfassungsvorrichtung, einer Haltung des Benutzers oder einer Abfolge von Bewegungen des Benutzers bezogen auf den Bildsensor der Bilderfassungsvorrichtung oder zur Befolgung eines Protokolls, das vom Endgerät präsentiert wird und vom Träger auszuführen ist, um ein bestätigtes Bild zu erhalten, erteilt.

5. Verfahren nach einem der vorhergehenden Ansprüche, wobei:
- während des Wiederholens der Schritte a) bis e) die Korrekturanweisungen, die vom Fernassistenzzentrum (22) erteilt werden, derart sind, dass die Nachrichten, die dem Benutzer vermittelt werden, diesen dazu veranlassen, eine Reihe von Augenfixierungen oder Augenverfolgungen vorzunehmen und/oder eine Reihe von Haltungen einzunehmen, in denen die Frankfurter Horizontale und/oder die Sagittalebene des Benutzers in einem vorgegebenen Winkel bezogen auf die optische Achse der Bilderfassungsvorrichtung ausgerichtet sind, oder ein Protokoll zu befolgen und auszuführen, das vom Endgerät präsentiert wird, um ein bestätigtes Bild zu erhalten;
- der Schritt a) das Aufnehmen, durch die Bilderfassungsvorrichtung (4), einer Videosequenz des Benutzers in der Reihe von Haltungen umfasst, die durch Fernassistenz bestätigt wird.

6. Verfahren nach dem vorhergehenden Anspruch, wobei die Reihe von Haltungen des Benutzers vor der Bilderfassungsvorrichtung (4) derart ist, dass die Frankfurter Horizontale und die Sagittalebene des Benutzers mit der optischen Achse der Bilderfassungsvorrichtung einen Winkel unter einer vorgegeben Schwelle bilden, und wobei bei Schritt g) ein Interpupillarabstand oder ein halber Interpupillarabstand berechnet wird.

7. Verfahren nach einem der vorhergehenden Ansprüche, wobei bei Schritt d) die Audio-Video-Fernassistenz das Kommunizieren von Neuskalierungsanweisungen an den Benutzer umfasst, aufweisend das Erfassen mindestens eines Bilds eines Elements bekannter Abmessung, das vorzugsweise in der Nähe der Augen des Benutzers angeordnet ist, oder das Erfassen eines Bilds, das für den Interpupillarabstand eines Benutzers repräsentativ ist, dessen Interpupillarabstand zuvor bekannt ist, oder ein Messen des bekannten Leseabstands, um die Konvergenz zu korrigieren, oder auch das Erfassen eines Bilds eines Gestells mit mindestens einer vorgegebenen geometrischen Abmessung.

8. Verfahren nach einem der vorhergehenden Ansprüche, wobei d) der Audio-Video-Fernassistenz die folgenden Unterschritte umfasst:
- einen Schritt des Fernübertragens eines Videostroms oder eines Bilds, das dem Fernassistenzzentrum bekannt ist, auf die grafische Schnittstelle des Endgeräts des Benutzers,
- einen Schritt des Erfassens eines Bilds des Gesichts oder wenigstens eines Auges des Benutzers in Reaktion auf diesen Videostrom oder dieses bekannte Bild.

9. Verfahren nach einem der vorhergehenden Ansprüche, wobei der Schritt g) eine Überprüfung eines Werts eines geometrischen Einstellungsparameters eines Brillengestells durch Vergleichen des bestimmten Werts mit einem Referenzwert aufweist.

10. Verfahren nach einem der vorhergehenden Ansprüche, wobei der Schritt g) ein Messen des Leseabstands oder des Absenkens des Kopfes / der Blickrichtung aufweist.

11. Verfahren nach einem der vorhergehenden Ansprüche, wobei:
- bei Schritt a) während des Erfassens mindestens eines Bilds oder einer Videosequenz der Benutzer mit einem Brillengestell ausgerüstet ist;
- die Nachrichten, die vom Endgerät gesendet werden, ein Führen des Benutzers in mindestens eine erste Haltung, bei der die Sagittalebene mit der Achse der Bilderfassungsvorrichtung ausgerichtet ist, und mindestens eine zweite Haltung, bei der die Sagittalebene zur Achse der Bilderfassungsvorrichtung einen Winkel ungleich null bildet;
- die Bilder oder Videosequenzen in diesen zwei Haltungen bestätigt werden.

12. Verfahren nach einem der vorhergehenden Ansprüche, umfassend einen zusätzlichen Schritt des Auswählens einer Sprache für den Benutzer aus einer Mehrzahl von Sprachen, und wobei, wobei eine Menge von Nachrichten zur Führung des Benutzers und zur Bestätigung gespeichert sind, jede Nachricht elektronisch mit ihrer audiovisuellen Implementierung in jeder der Sprachen der Mehrzahl von Sprachen verknüpft ist, wobei die Nachricht dem Benutzer durch das Endgerät entsprechend der Anweisung, die durch das Fernassistenzzentrum erteilt wird, und in der Sprache, die vom Benutzer auswählt wird, vermittelt wird.

13. Verfahren nach einem der vorhergehenden Ansprüche, wobei der Schritt c) des Verarbeitens, durch das Fernassistenzzentrum (22), des mindestens einen Bilds oder der mindestens einen Videosequenz, das/die bei Schritt b) und/oder Schritt d) des Fernkommunizieren der Korrektur- oder Bestätigungsanweisung vom Fernassistenzzentrum (22) an das elektronische Endgerät (2) des Benutzers übertragen wird, von einem automatisierten System durchgeführt wird.

14. Verfahren nach einem der vorhergehenden Ansprüche, wobei der Schritt c) des Verarbeitens, durch das Fernassistenzzentrum (22), des mindestens einen Bilds oder der mindestens einen Videosequenz, das/die bei Schritt b) und/oder Schritt d) des Fernkommunizieren der Korrektur- oder Bestätigungsanweisung vom Fernassistenzzentrum (22) an das elektronische Endgerät (2) des Benutzers übertragen wird, von einem Optiker (11) durchgeführt wird, der über eine digitale Signatur verfügt.

## Claims

1. A method for determining at least one value of a personalization parameter of a piece of vision-correcting equipment for a user (1), employing an electronic terminal (2) comprising a graphical interface (3), an image capturing apparatus (4) comprising a single image sensor and audiovisual communication means (5), the method comprising the following steps:
a) capturing at least one image or video sequence of the user by means of the image capturing apparatus (4);
b) communicating said at least one image or video sequence captured in step a) to a remote-assistance center (22) located remotely from the electronic terminal (2), the remote-assistance center (22) comprising at least one checking terminal (12);
c) checking processing, by the remote-assistance center (22), of said at least one image or video sequence transmitted in step b), in order to deduce therefrom, on account of a position, a posture or a sequence of movements of the user in front of the image capturing apparatus, a captured image or video sequence correction or validation instruction;
d) communicating from the remote-assistance center (22) to the electronic terminal (2) of the user said correction or validation instruction;
e) the electronic terminal (2) displaying or emitting a video or audio message informing the user of the validation of the image or video sequence capture or requesting the user to position or move himself relative to the image capturing apparatus in accordance with the correction instruction, step e) comprising a protocol of alignment of the Frankfurt plane of the head of the user with the axis of the image capturing apparatus, the protocol consisting in a competent person of the remote-assistance center requesting the user to capture a first front-on image and then comprising a request for the user to rotate his head about a horizontal axis until the Frankfurt plane is aligned parallel to the axis of the image capturing apparatus and then comprising a request for the user to rotate his head to the right or left in order to allow the competent person to see the tragions and determine a measurement posture wherein the axis of the image capturing apparatus is located in the Frankfurt plane of the user;
f) reiterating the preceding steps to comply with and execute the protocol of alignment of the Frankfurt plane parallel to the axis of the image capturing apparatus until a validated image or video sequence in said measurement posture is obtained ; and
g) determining at least one value of said personalization parameter depending on said captured and validated at least one image or video sequence in said measurement posture, said at least one personalization parameter comprising an interpupillary distance or left or right monocular pupillary distance parameter.

2. The method as claimed in claim 1, in which said at least one personalization parameter comprises one of the following parameters:
- a user face shape parameter;
- a user posture parameter;
- a dynamic behavioral parameter of the user;
- a centration parameter of a corrective lens in a spectacle frame;
- a parameter characterizing the position of the pupillary centers or CRE of the eyes in a frame of reference associated with the frame;
- a vertex parameter, for example a lens-eye distance;
- a parameter characterizing the position or inclination of the frame or of the piece of equipment or of the lens in a face or environment frame of reference, for example a face form angle or a pantoscopic angle; and
- adjustment parameters of the frame in a face frame of reference.

3. The method as claimed in one of the preceding claims, in which, in step d), the remote-assistance center (22) delivers a validation instruction if said at least one image or video sequence is good enough to at least contribute to determining the value of the sought personalization parameter, or delivers a correction instruction in the contrary case, respectively.

4. The method as claimed in one of the preceding claims, in which in step d), the remote-assistance center (22) delivers a correction instruction to correct a centration position of the eyes in front of the image capturing apparatus, a posture of the user, a sequence of movements of the user relative to the image sensor of the image capturing apparatus, or compliance with a protocol presented by the terminal and to be executed by the wearer in order to obtain a validated image.

5. The method as claimed in one of the preceding claims, in which:
- during the reiteration of steps a) to e), the correction instructions delivered by the remote-assistance center (22) are such that the messages delivered to the user lead him to adopt a series of ocular fixations or ocular pursuits and/or a series of postures in which the Frankfurt plane and/or sagittal plane of the user are oriented at a predefined angle to the optical axis of the image capturing apparatus, or to comply with and execute a protocol presented by the terminal in order to obtain a validated image; and
- step a) comprises the image capturing apparatus (4) taking a video recording of a video sequence of the user in the remotely validated series of postures.

6. The method as claimed in the preceding claim, in which the series of postures of the user in front of the image capturing apparatus (4) is such that the Frankfurt plane and the sagittal plane of the user make to the optical axis of the image capturing apparatus an angle smaller than a predefined threshold and in which in step g), an interpupillary distance or a monocular pupillary distance is calculated.

7. The method as claimed in one of the preceding claims, in which in step d), the audio-video remote-assistance comprises communicating to the user rescaling instructions including capturing at least one image of an element of known size, preferably placed in proximity to the eyes of the user, or capturing an image representative of the interpupillary distance of a user whose interpupillary distance is already known, or measuring a known reading distance in order to correct convergence, or even capturing an image of a frame having at least one predefined geometrical dimension.

8. The method as claimed in one of the preceding claims, in which step d) of audio-video remote-assistance comprises the following substeps:
- a step in which the remote-assistance center transmits to the graphical interface of the terminal of the user a video stream or a known image; and
- a step of capturing an image of the face or at least one eye of the user in response to this video stream or this known image.

9. The method as claimed in one of the preceding claims, in which step g) comprises verifying a value of a geometric adjustment parameter of a spectacle frame by comparing said determined value with a reference value.

10. The method as claimed in one of the preceding claims, in which step g) comprises measuring reading distance or gaze direction/by how much the head is lowered.

11. The method as claimed in one of the preceding claims, in which:
- in step a) during the capture of at least one image or video sequence, the user is equipped with a spectacle frame;
- the messages emitted by the terminal allow the user to be guided into at least one first posture in which the sagittal plane is aligned with the axis of the image capturing apparatus and at least one second posture in which the sagittal plane makes a nonzero angle to the axis of the image capturing apparatus; and
- the images or video sequences are validated in these two postures.

12. The method as claimed in one of the preceding claims, comprising an additional step of selecting a language for the user from a plurality of languages, and in which, a set of validation and user guidance messages being recorded, each message is informationally tied to its audiovisual implementation in each of the languages of the plurality of languages, the message being delivered by the terminal to the user in correspondence with the instruction delivered by the remote-assistance center and in the language selected by the user.

13. The method as claimed in one of the preceding claims, in which step c) of processing, by the remote-assistance center (22), of said at least one image or video sequence transmitted in step b) and/or step d) of communicating, from the remote-assistance center (22) to the electronic terminal (2) of the user, said correction or validation instruction, is carried out by an automated system.

14. The method as claimed in one of the preceding claims, in which step c) of processing, by the remote-assistance center (22), of said at least one image or video sequence transmitted in step b) and/or step d) of communicating, from the remote-assistance center (22) to the electronic terminal (2) of the user, said correction or validation instruction, is carried out by an optician (11) possessing a digital signature.
